# EUROPEAN PATENT SPECIFICATION

(11) **EP 3 856 295 B1**
(45) Date of publication and mention of the grant of the patent: **08.03.2023**
(21) Application number: 19778692.4
(22) Date of filing: 25.09.2019
(51) Int. Cl.: A61M 5/315, A61M 5/24, A61M 5/31

(54) **INJECTION DEVICE**
INJEKTIONSVORRICHTUNG
DISPOSITIF D'INJECTION

(30) Priority: 26.09.2018 EP 18196934
(43) Date of publication of application: 04.08.2021
(73) Proprietor: Galderma S.A., 6330 Cham (CH)
(72) Inventor: KARLSSON, Henrik, 749 52 Grillby (SE); ÖHRLUND, Åke, 752 60 Uppsala (SE)
(74) Representative: Impact Intellectual Property LLP
(86) International application number: PCT/IB2019/058149
(87) International publication number: WO 2020/065563

(56) References cited:
- WO-A1-2008/101829
- WO-A2-2016/186965
- US-A- 5 782 633
- US-A1- 2005 165 363

## Description

### FIELD OF THE INVENTION

The present invention relates to an injection device for delivering liquid compositions.

### BACKGROUND OF THE INVENTION

Injection devices are commonly used for injection of all kinds of liquid substances. However, in some areas of application, such as for example fillers of different kinds, the viscosity of the substances has gradually increased over the years and, also, the gauge size of needles has become thinner. This has a major impact on the force required for extruding the substances. High viscosity of a substance and thin gauge sizes of a needle generally requires application of a high force to the injection device in order to inject the substance. This can be tiresome for a person operating the injection device, and particularly so when the operation time is lengthy. A need for an injection device requiring a reduced force for injection of high viscosity substances therefore exists.

US9446200 discloses a drug delivery system that facilitates high pressure medication injections. The drug delivery system comprises a system of levers and gears to produce a sufficient force for an intradermal injection without increasing the input force required from the user. This drug delivery system is rather complex comprising multiple components which interact to inject a certain dose of a substance. The drug delivery system is also rather bulky.

US 5 782 633 A relates to an applicator for a dental compound, whose elongate implement body is or can be connected at the front end to a syringe containing the dental compound and has a ram for advancing a plunger contained in the syringe, which plunger can be pushed forward by means of a pusher rod protruding out of the rear end of the implement body. The ram and the pusher rod are each connected to a rack between which racks a gearwheel/pinion pair is arranged, which brings about a gear reduction from the pusher rod to the ram.

US 2005/165363 A1 relates to a medication dispensing apparatus having a gear set. The gear set has a first pinion in meshed engagement with a rack of the plunger, and a second pinion in meshed engagement with a rack of a drive member of the apparatus. The gear set operatively interconnects a plunger and the drive member such that after the plunger is moved relative to the housing in a proximal direction to prepare the apparatus for injection, the plunger, when manually pushed back toward the housing, causes the drive member to advance in a distal direction to force medication through an outlet, typically provided with an injection needle, at the distal end of the apparatus.

WO 2016/186965 A2 relates to an injection device having a drive member with first and second gear patterns that are fixed relative to each other. The drive member is rotatable and translatable in first and second opposing axial directions. A plunger expels medicament from a cartridge when it is translated in an advancing direction. A dosage indicator indicates a set dosage. A first gearing arrangement operably couples the first gear pattern with the plunger wherein rotation of the drive member in a first direction translates the drive member in a first axial direction without movement of the plunger and translation of the drive member in the opposite axial direction drives the plunger in the advancing direction. A second gearing arrangement operably couples the second gear pattern with the dosage indicator and moves the dosage indicator to indicate an increase in the set dosage when the drive member is translated in the first axial direction.

WO 2008/101829 A1 relates to an injection device comprising a dose setting mechanism and injection means. The injection device comprises a lead screw which is adapted to perform a rotational movement when a dose setting means is rotated during dose setting, and which is adapted to cooperate with a piston of a cartridge in order to cause a set dose of drug to be injected from the cartridge. The lead screw is connected to the injection means via a worm gear connection in such a manner that linear movement of the injection means causes linear movement of the lead screw. Thus, the dose setting means and the injection means are connected via the lead screw and the worm gear connection. Thereby the dose setting mechanism and the injection mechanism are integrated into each other by, at least partly, using the same components.

### SUMMARY OF THE INVENTION

It is an object of the present invention to provide an improved injection device which reduces or eliminates at least the above mentioned drawbacks. This object is achieved by an injection device according to the present invention as defined in claim 1 of the appended claims. Preferred embodiments of the present invention are defined in the dependent claims.

To better address this concern, in a first aspect of the invention there is presented an injection device comprising a drive mechanism, the drive mechanism comprising a drive rod being axially moveable, a plunger rod being axially moveable, and a gear assembly. The gear assembly is arranged such that an axial movement of the drive rod generates an axial movement of the plunger rod. The gear assembly allows for a force transmission between the drive rod and the plunger rod, such that a force applied to the drive rod is transmitted and reinforced by the gear assembly to the plunger rod. This facilitates expelling viscous substances from an injection device without having to apply an excessive input force to the drive rod. According to the invention, the drive mechanism is arranged at a finger grip of the injection device, wherein the finger grip is releasably arranged.

In accordance with an embodiment of the injection device, the axial movement of the drive rod generates an axial movement of the plunger rod in the same direction. This allows for the injection device to be operable as a traditional handheld syringe, expelling a substance contained therein by moving the drive rod in an axial direction of the injection device towards the patient receiving the expelled substance.

In accordance with an embodiment of the injection device, the drive rod comprises a grooved surface and the plunger rod comprises a grooved surface. The grooved surfaces of the drive rod and the plunger rod allows entering in a simple engagement with the gear assembly. The grooves are preferably arranged along the entire longitudinal extension of the drive rod and the plunger rod, respectively. In some embodiments, any one of the drive rod and the plunger rod may be provided with grooved surface portions while other surface portions may not be grooved. As an example, the drive rod may have a rectangular cross-section, thus having four outer longitudinal surfaces out of which at least one is grooved. Other shapes of the drive and plunger rod cross-sections are however conceivable, such as round or square, and may under some circumstances be preferable, within the concept of the present invention.

In accordance with an embodiment of the injection device, the gear assembly comprises a drive cogwheel engaged with the drive rod, and a plunger cogwheel engaged with the plunger rod, the drive cogwheel and the plunger cogwheel being connected to each other. The drive cogwheel will thus enter in rotation when the drive rod is moved in an axial direction. The connection between the drive cogwheel and the plunger cogwheel is such that a rotation of the drive cogwheel generates a rotation of the plunger cogwheel. The rotation of the plunger cogwheel will, in turn, generate an axial movement of the plunger rod. The drive cogwheel may be connected to the plunger cogwheel by means of a cogwheel engagement, in which the cogged outer circumferences of the drive cogwheel and the plunger cogwheel respectively are engaged, whereby a rotation of the drive cogwheel in the clockwise direction generates a rotation of the plunger cogwheel in the counter-clockwise direction. In another embodiment, the drive cogwheel is connected to the plunger cogwheel by engaging side surfaces. In this embodiment, the drive cogwheel and the plunger cogwheel are arranged on the same axis around which they can rotate. The engagement of the respective side surfaces of the drive cogwheel and the plunger cogwheel is such that rotation of the drive cogwheel in the clockwise direction generates a rotation of the plunger cogwheel in the same direction.

In accordance with an embodiment of the injection device, a circumference of the drive cogwheel is smaller than a circumference of the plunger cogwheel, the circumference of the plunger cogwheel being in engagement with the circumference of the drive cogwheel. A small circumference of the drive cogwheel allows for a compact injection device. The engagement of the circumference of the drive cogwheel with the circumference of the plunger cogwheel, allows arranging the drive rod and the plunger rod at different sides of the axis around which the plunger cogwheel rotates. This provides for an advantageous centralised distribution of the drive rod and the plunger rod, without the two parts interfering with each other. In this embodiment, the axial movement of the plunger rod, in engagement with the plunger cogwheel, generated by the axial movement of the drive rod, in engagement with the drive cogwheel, will be in the same direction as the axial movement of the drive rod.

In accordance with an embodiment of the injection device, the plunger cogwheel comprises a first wheel element engaged with the drive cogwheel, and a second wheel element engaged with the plunger rod. The first and second elements of the plunger cogwheel can be adapted to the force transmission needed in the injection device by adapting their respective diameters. By providing a first wheel element which has a larger diameter than the second wheel element, provided it rotates at the same angular rate as the second wheel element, a transmission ratio between the drive rod and the plunger rod of 1:X is achieved, where X is greater than 1. The diameters of the first and second wheel elements of the plunger cogwheel may generally be adapted to achieve a force transmission ratio between the drive rod and the plunger rod within the range from 1:2 to 1:4, such as for example 1:3.

In accordance with an embodiment of the injection device, the first wheel element is rotatable with respect to the second wheel element in one direction only. Due to the force transmission from the drive rod to the plunger rod through the gear assembly, as the drive rod is moved a certain axial distance, a movement of the plunger rod of a shorter axial distance is generated. The first wheel element being rotatable with respect to the second wheel element in one direction only allows returning the drive rod to its initial position by moving it in an axial direction opposite to a substance expelling direction, in which the movement causes substance to be expelled from the injection device, without also moving the plunger rod. Thereby the length of the drive rod does not need to be longer than that of the plunger rod.

In accordance with an embodiment of the injection device, the plunger cogwheel comprises a first peripheral cog portion arranged at the first wheel element, and a second peripheral cog portion arranged at the second wheel element. The first and second peripheral cog portions allow the first and second wheel elements to engage with the cogged or grooved surfaces of the drive cogwheel and the plunger rod, respectively.

In accordance with an embodiment of the injection device, the first peripheral cog portion has a circumference that is larger than a circumference of the second peripheral cog portion.

In accordance with an embodiment of the injection device, the drive cogwheel and the plunger cogwheel are arranged on the same axis. In this embodiment, the arrangement of the drive cogwheel and the plunger cogwheel is such that a movement of the drive rod in an axial direction generates a rotation of both cogwheels in the same direction and at the same angular rate, whereby a movement of the plunger rod in an axial direction is generated. This allows arranging the drive rod and the plunger rod at the same side of the axis on which the drive cogwheel and the plunger cogwheel are arranged and around which they rotate, thereby providing a compact injection device.

In accordance with an embodiment of the injection device, a circumference of the drive cogwheel is larger than a circumference of the plunger cogwheel. This enables a transmission between the drive rod and the plunger rod as the drive rod is moved axially, such that the plunger rod is axially moved7 with a force that is higher than that induced in the drive rod for causing its axial movement.

In accordance with an embodiment of the injection device, the drive cogwheel is rotatable with respect to the plunger cogwheel in one direction only. This allows moving the drive rod axially in one, non-substance expelling, direction without generating a movement of the plunger cogwheel and, consequently, of the plunger rod.

In accordance with an embodiment of the injection device, the drive rod has an idle position and the injection device further comprises a return spring arranged to force the drive rod towards the idle position. When no pressure is exerted on the drive rod it is in the idle position and the return spring is in an extended state. When pressure is applied to the drive rod to move it axially in the substance expelling direction, the return spring is compressed. Therefore, when the pressure exerted on the drive rod is removed therefrom, the return spring will spring back to its extended state, whereby the drive rod will be forced to move axially in a direction opposite to the direction in which the pressure was applied. To apply an injection with the injection device, the drive rod can therefore be operated in a manner similar to that of a click-pen, being pressed in an axial direction several times in order to inject a substance into a patient.

According to the invention, the drive mechanism is arranged at a finger grip of the injection device. Arranging the drive mechanism at a finger grip of the injection device allows for an injection device which is easy and ergonomic for the user to operate.

According to the invention, the finger grip is releasably arranged. This allows the finger grip comprising the drive mechanism to be reused for several injection devices. This also allows adapting the finger grip to the desired force transmission, as finger grips comprising drive mechanisms providing different transmission ratios between the drive rod and the plunger rod may be provided, between which the operator of the injection device may choose as a function of the viscosity of the substance to inject or of the gauge size of the needle.

### BRIEF DESCRIPTION OF THE DRAWINGS

The invention will now be described in more detail and with reference to the appended drawings in which:
Fig. 1a is a perspective view of an embodiment of the injection device according to the present invention;
Fig. 1b is an exploded view of an embodiment of the injection device according to the present invention;
Fig. 2a is a cutaway view of an embodiment of the injection device according to the present invention;
Fig. 2b is a schematic view of an embodiment of a drive mechanism of the injection device according to the present invention;
Figs. 3a-b show schematic perspective views of an embodiment of a gear assembly of the drive mechanism of the injection device according to the present invention; and
Fig. 4 shows a cutaway view of an embodiment of the injection device according to the present invention.

### DESCRIPTION OF EMBODIMENTS

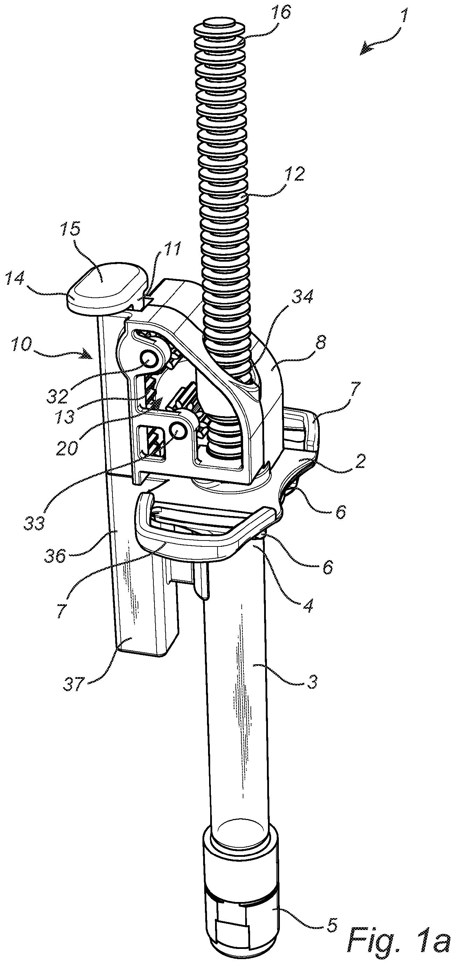

Referring to Figs. 1a-b, there is provided an injection device 1 comprising a finger grip 2 and a cartridge 3. The cartridge 3 comprises a proximal end 4 and a distal end 5, the distal end 5 being that oriented towards a patient receiving an injection from the injection device 1. The distal end 5 of the cartridge 3 thus comprises a portion adapted to hold an injection needle, not shown. The cartridge 3 further comprises a plunger (not shown) which upon application of a pressure thereto in a direction towards the distal end 5 of the cartridge, which hereinafter also will be referred to as a substance expelling direction, acts to expel a substance contained in the cartridge 3.

Generally, any longitudinal part of the injection device 1 which extends in a direction parallel to the cartridge 3 will be considered to comprise a distal end, being that arranged closest to the distal end 5 of the cartridge 3, and a proximal end, being that arranged furthest away from the distal end 5 of the cartridge 3. Similarly, a longitudinal or axial movement in a distal direction shall be considered to represent a movement towards the distal end 5 of the cartridge 3, and thus, as a substance expelling direction.

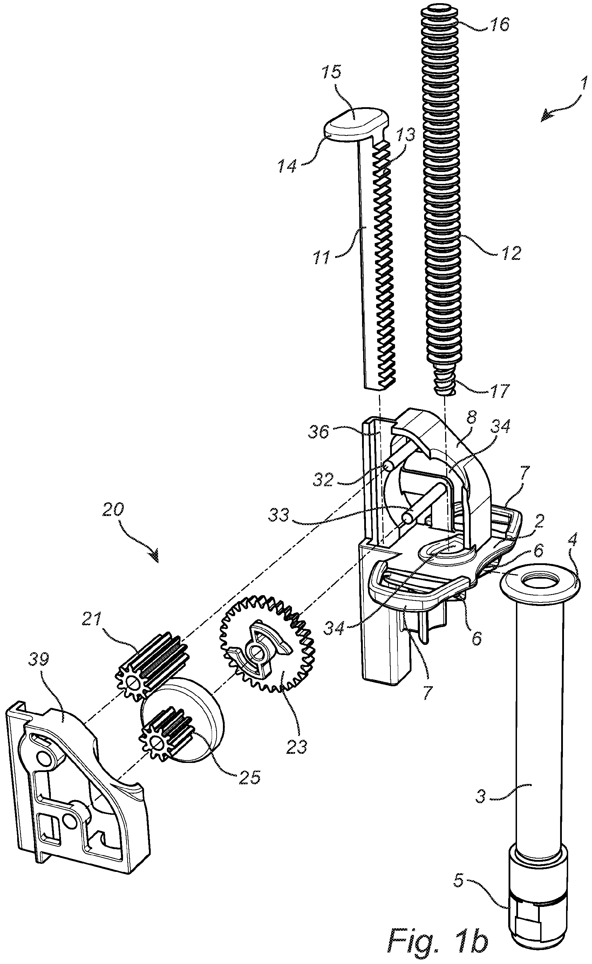

The proximal end 4 of the cartridge 3 is arranged in engagement with the finger grip 2. In the exemplifying embodiment of Fig. 1, the finger grip 2 is provided with tracks 6 for receiving and holding the proximal end 4 of the cartridge 3, see Fig. 1b. Other ways of arranging the cartridge 3 in engagement with the finger grip 2 are also conceivable within the concept of the present invention.

The finger grip 2 comprises two oppositely extending flanges 7, which extend in a direction perpendicular to the longitudinal direction of the cartridge 3, and which are arranged for providing a finger grip for a person operating the injection device 1. The finger grip 2 further comprises a housing 8 which houses a drive mechanism 10.

The drive mechanism 10 comprises a drive rod 11, a plunger rod 12 and a gear assembly 20. The drive rod 11 is elongated and has a rectangular cross-section. Other cross-sections, such as square or circular, are also possible within the concept of the present invention. The drive rod 11 has at least one grooved elongated surface 13. The grooved surface 13 is arranged to engage with the gear assembly 20, as will be described in more detail further on. At a proximal end of the drive rod 11, a pressure application portion 14 is arranged. The pressure application portion 14 comprises an upper end surface 15, onto which pressure from an operator can be applied, for example by using the thumb, in order to push the drive rod 11 in a direction towards a distal end thereof.

The plunger rod 12 is elongated and has a circular cross-section. However, other cross-sections, such as for example rectangular or square, are also conceivable within the concept of the present invention. The plunger rod 12 has a grooved elongated surface 16, in this embodiment extending around the complete extension of the plunger rod 12. In an alternative embodiment in which the plunger rod 12 has a rectangular or square cross-section, at least one elongated surface of the plunger rod 12 is grooved. The plunger rod 12 is arranged such that the grooved surface 16 engages the gear assembly 20 as will be described in more detail further on.

At a distal end of the plunger rod 12, a plunger engagement portion 17 is provided, see Fig. 1b. In the shown embodiment, the plunger engagement portion 17 comprises an outer thread, adapted to engage with an inner thread of a plunger arranged in the cartridge 3. Considering the threaded engagement of the plunger rod 12 and the plunger, it is preferable that the plunger rod 12 of this embodiment has a circular cross-section with grooves extending around its entire extension, such that the grooves are accessible for engaging the gear assembly 20 independently of the angular position of the plunger rod 12. Other engagements between the plunger rod 12 and the plunger are, however, also conceivable within the concept of the present invention, such as for example a snap-in engagement.

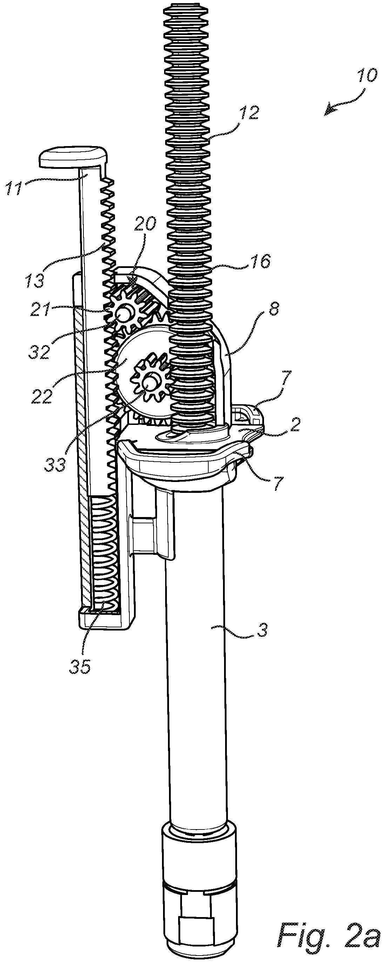

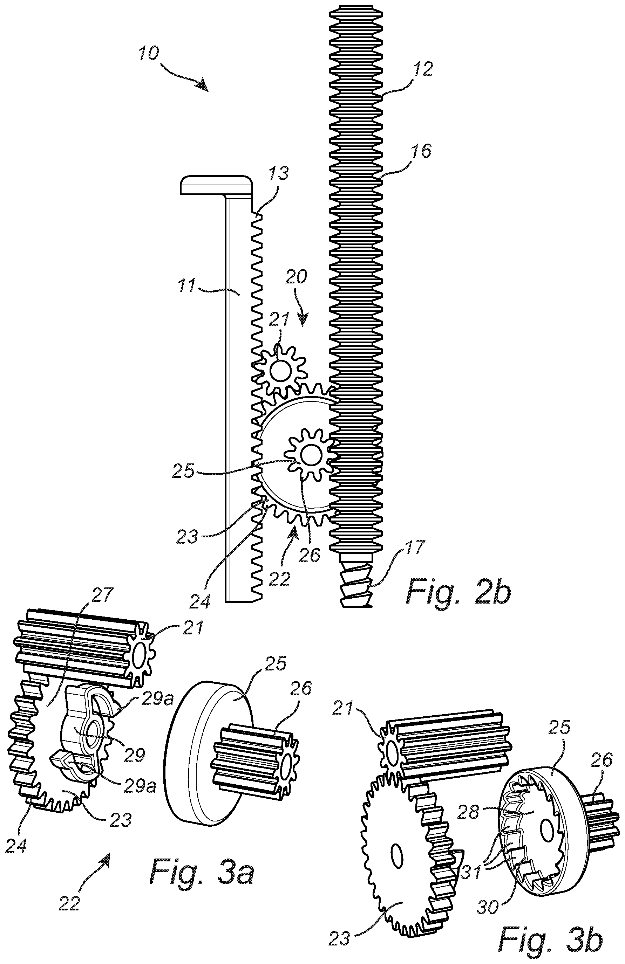

The gear assembly 20 is arranged in engagement with the drive rod 11 and the plunger rod 12. As shown in Figs. 2a-b, the gear assembly 20 comprises a drive cogwheel 21 engaged with the grooved surface 13 of the drive rod 11. The gear assembly 20 further comprises a plunger cogwheel 22 engaged with the grooved surface 16 of the plunger rod 12. The plunger cogwheel 22 of this embodiment comprises a first wheel element 23 with a first peripheral cog portion 24 which engages the drive cogwheel 21, see Fig. 2b. The circumference of the first peripheral cog portion 24 is here larger than the circumference of the drive cogwheel 21, allowing a compact arrangement of the drive mechanism 10. Providing a drive cogwheel 21 with a circumference larger than that of the first peripheral cog portion 24 is also conceivable, although it would result in a less space efficient drive mechanism 20. The plunger cogwheel further comprises a second wheel element 25 with a second peripheral cog portion 26 which engages the grooved surface 16 of the plunger rod 12. The circumference of the first peripheral cog portion 24 is larger than the circumference of the second peripheral cog portion 26, allowing a force transmission between the drive rod and the plunger rod that is larger than 1. The first and second wheel elements 23, 25 are further centralised on a common axis 33 around which they are rotatable.

The first and second wheel elements 23, 25 each have engaging side surfaces 27, 28, see Figs. 3a-b. In this exemplifying embodiment, the engaging side surface 27 of the first wheel element 23 comprises a protruding portion 29, protruding in an axial direction of the plunger cogwheel 22 and extending in a circular direction thereof. The engaging side surface 28 of the second wheel element 25 comprises a recessed portion 30, arranged to receive and engage with the protruding portion 29 of the first wheel element 23. The recessed portion 30 is provided with hook receiving wedge shaped recesses 31 around an inner circumference thereof, so arranged to engage hook portions 29a of the protruding portion 29 of the first wheel element 23. The hook portions 29a of the protruding portion 29 thereby prevent the first wheel element 23 from rotating with respect to the second wheel element 25 in one direction. The wedge shaped recesses 31 are shaped such that the inclination of one wedge side of each wedge is larger than the inclination of the other wedge side of each wedge. Furthermore, the hook portions 29a are resilient. This allows the hook portions 29a of the protruding portions 29 to slide along the less inclined wedge side from one wedge to another upon rotation in an opposed direction such that the first wheel element 23 rotates with respect to the second wheel element 25. In this embodiment, the resilience of the hook portions 29a is obtained by providing the protruding portion 29 in a rather flexible material, such as a plastic material, thereby enabling the sliding of the hook portions 29a along the less inclined wedge side from one wedge to another.

Referring back to Figs. 1 a-b, the housing 8 is provided with a drive axis 32 and a plunger axis 33. The drive axis 32 is arranged to rotatably carry the drive cogwheel 21, and the plunger axis 33 is arranged to rotatably carry the plunger cogwheel 22. The plunger and drive axes 32, 33 are arranged such that the drive cogwheel 21 and the plunger cogwheel 22 enter in a cogwheel engagement when respectively mounted thereon. More particularly, the plunger and drive axes 32, 33 are arranged such that the drive cogwheel 21 and the first wheel element 23 of the plunger cogwheel 22 enter in a cogwheel engagement when respectively mounted thereon. In the exemplifying embodiment shown, the drive axis 32 is arranged proximally of the plunger axis such that the drive cogwheel 21 engages the plunger cogwheel 22 at a proximal portion of the plunger cogwheel 22. The skilled person understands, however, that arranging the drive axis for example distally of the plunger axis such that the drive cogwheel 21 and the plunger cogwheel 22 engage at a distal portion of the plunger cogwheel 22 also is conceivable within the concept of the present invention.

The housing 8 further comprises upper and lower apertures 34 through which the plunger rod 12 is arranged and can travel in an axial direction. A guiding track 36 is further provided for the drive rod 11 for guiding the drive rod 11 in a longitudinal axial direction. At the distal end 37 of the guiding track 36, a return spring 35 is provided extending in a proximal direction. A proximal end of the spring 35 is arranged to support the distal end of the drive rod 11 when arranged in the guiding track 36.

Finally, the housing 8 comprises a closing wall portion 39 which secures the drive mechanism 10 at the finger grip 2.

When the injection device comprising the drive mechanism 10 is mounted and no pressure is applied to the drive rod 11, the drive rod 11 is in an idle position, in which it protrudes to a maximum from the guiding track 36 in the proximal direction. In the idle position, the gear assembly 20 does not rotate and the plunger rod 12 does not move. To operate the injection device, the drive rod 11, arranged in the guiding track 36, is pressed in a distal direction, whereby the drive cogwheel 21 enters in rotation in a counter-clockwise direction, as seen from the side shown in Figs. 2a-b. This drives the plunger cogwheel 22, with first and second wheel elements 23, 25, to rotate in a clockwise direction. The second wheel element 25, being engaged with the plunger rod 12, thereby forces the plunger rod 12 to travel in a distal direction of the injection device 1, and of the cartridge 3, causing the plunger to move in the distal direction thereof such that a substance contained in the cartridge 3 is expelled.

Due to the arrangement of the gear assembly 20, having differently sized circumferences which engage the drive rod 11 and the plunger rod 12, respectively, there will be a force transmission between the drive rod 11 and the plunger rod 12. Typically, the force transmission from the drive rod 11 to the plunger rod 12 can be of a factor of 2 to 4, such as for example 3. As a result, a viscous substance contained in the cartridge 3 can be expelled therefrom by means of the injection device 1 without having to apply a considerable force to the drive rod 11. Also, since the drive rod 11 will travel a larger distance with respect to the plunger rod when pressure is applied thereto, the injection device 1 is provided with the return spring 35 at the bottom of the guiding track 36 of the finger grip 2. When the drive rod 11 has been pressed down and is released, the return spring 35 will spring back to its extended state, forcing the drive rod 11 towards the idle position. From the idle position, the drive rod 11 can be pressed anew towards the distal end thereof to continue generating an axial movement of the plunger rod 12 towards the distal end of the cartridge 3.

When the drive rod 11 is forced by the return spring 35 to travel in a proximal direction, the drive cogwheel 21 enter in rotation in a clockwise direction, as seen from the side view of Fig. 2a, causing the first wheel element 23 of the plunger cogwheel 22 to rotate in a counter-clockwise direction. The less inclined wedge sides of the wedge shaped recesses 31 of the second wheel element 25 here allows the resilient hook portions 29a of the first wheel element 23 to slide there along such that the first wheel element 23 rotates with respect to the second wheel element 25. Thereby, the second wheel element 25 remains in position and the plunger rod 12 does not move. When the drive rod 11 is moved in an axial direction towards the distal end 37 of the guiding track 7 anew, the hook portions 29a of the protrusions 29 of the first wheel element 23 will engage the hook receiving wedge shaped recesses 31 of the recessed surface 30 of the second wheel element 25 such that the second wheel element 25 rotates with the first wheel element 23 in a clockwise direction as viewed in Fig. 2a, thereby generating an axial movement of the plunger rod 12 towards the distal end of the cartridge 3.

The drive rod 11 and plunger rod 12 are in the above described embodiment arranged at different sides of the drive and plunger axes 32, 33 requiring the drive cogwheel 21 to act on the plunger cogwheel 22 in order to generate an axial movement of the plunger rod 12 in the same direction as the axial movement of the drive rod 11 when the latter is pressed towards its distal end.

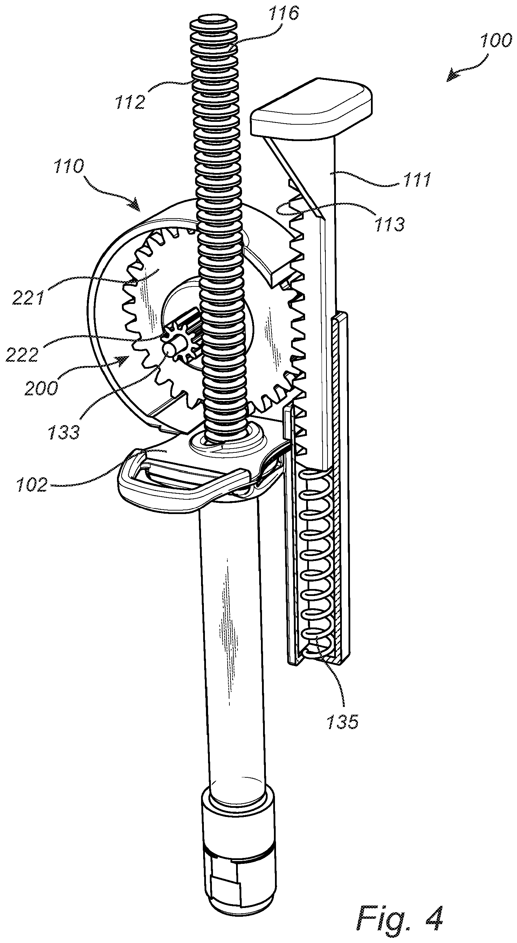

According to another embodiment of the injection device 100, and with reference to Fig. 4, the drive rod 111 and the plunger rod 112 are arranged at the same side of an axis 133 at which the gear assembly 200 is arranged, and around which the gear assembly 200 is rotatable. In this embodiment, the gear assembly 200 comprises a drive cogwheel 221 engaged with the drive rod 111, and a plunger cogwheel 222 engaged with the plunger rod 212. The drive cogwheel 221 and the plunger cogwheel 222 are connected to each other by means of engaging side surfaces, corresponding to the engaging side surfaces 27, 28 of the first and second wheel elements 23, 25 of the embodiment described with reference to Figs. 3a-b. The engaging side surface 27 of the drive cogwheel 221 comprises protruding portions 29 protruding in the axial direction of the drive cogwheel 221. The engaging side surface 28 of the plunger cogwheel 222 comprises a recessed surface 30 for receiving the protruding portions 29 of the drive cogwheel 221. The protruding portions 29 of the drive cogwheel 221 side surface are further provided with hook portions 29a which can engage hook receiving wedge shaped recesses 31 at the inner circumference of the recessed surface 30 of the plunger cogwheel 222. This allows the drive cogwheel 221 to rotate with respect to the plunger cogwheel 222 in one direction only. The circumference of the drive cogwheel 221 is larger than the circumference of the plunger cogwheel 222. As a consequence, when the drive rod 111 is pressed in an axial direction, an axial movement of the plunger rod 112 in the same direction will be generated with a force transmission. The size of the drive and plunger cogwheels 221, 222 can be adapted to meet the need of particular force transmissions. Generally, a force transmission of 2-5 times that induced in the drive rod 111 can be generated for the plunger rod 112, consequently reducing significantly the force needed to inject a viscous substance.

While the invention has been illustrated and described in detail in the drawings and foregoing description, such illustration and description are to be considered illustrative or exemplary and not restrictive. The invention is not limited to the disclosed embodiments.

For instance, the housing 8 of the embodiment shown in Figs. 1-2 can alternatively be embodied as having a partition between the bearing structures of the drive axis 32 and the plunger axis 33. Furthermore, the return spring 35 of this alternative embodiment is arranged in the partition to act on the bearing structure of the drive axis 32, such that the drive axis can be moved between an idle position and a drive position when applying a pressure to the plunger rod 11 in a substance expelling direction. In the idle position, the return spring 35 is extended, forcing the drive axis to move in a proximal direction such that the drive cogwheel 21 arranged thereat does not engage the plunger cogwheel 22 arranged at the plunger axis 33. As pressure is applied to the drive rod 11, the return spring is compressed and the drive axis moved to the drive position, in which the drive cogwheel 21 engages the plunger cogwheel 22. This embodiment allows the first and second wheel elements 23, 25 of the plunger cogwheel 22 to be embodied as one single part, or to be in a sealing engagement with each other such that no relative rotation there between is possible.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An injection device (1, 100) comprising:
a drive mechanism (10, 110), the drive mechanism comprising:
a drive rod (11, 111) being axially moveable;
a plunger rod (12, 112) being axially moveable; and
a gear assembly (20, 200),
wherein the gear assembly is arranged such that an axial movement of the drive rod generates an axial movement of the plunger rod,
wherein the drive mechanism (10, 110) is arranged at a finger grip (2, 102) of the injection device, and
**characterised in that** the finger grip (2, 102) is releasably arranged.

2. The injection device (1, 100) according to claim 1, wherein the axial movement of the drive rod (11, 111) generates an axial movement of the plunger rod (12, 112) in the same direction.

3. The injection device (1, 100) according to claim 1 or 2, wherein the drive rod (11, 111) comprises a grooved surface (13, 113) and the plunger rod (12, 112) comprises a grooved surface (16, 116).

4. The injection device (1, 100) according to any one of the preceding claims, wherein the gear assembly (20, 200) comprises a drive cogwheel (21, 221) engaged with the drive rod (11, 111), and a plunger cogwheel (22, 222) engaged with the plunger rod (12, 112), the drive cogwheel and the plunger cogwheel being connected to each other.

5. The injection device (1) according to claim 4, wherein a circumference of the drive cogwheel (21) is smaller than a circumference of the plunger cogwheel (22), the circumference of the plunger cogwheel being in engagement with the circumference of the drive cogwheel.

6. The injection device (1) according to claim 4 or 5, wherein the plunger cogwheel (22) comprises a first wheel element (23) engaged with the drive cogwheel (21), and a second wheel element (25) engaged with the plunger rod (12).

7. The injection device (1) according to claim 6, wherein the first wheel element (23) is rotatable with respect to the second wheel element (25) in one direction only.

8. The injection device (1) according to claim 6 or 7, wherein the plunger cogwheel (22) comprises a first peripheral cog portion (24) arranged at the first wheel element (23), and a second peripheral cog portion (26) arranged at the second wheel element (25), and
wherein the first peripheral cog portion (24) has a circumference that is larger than a circumference of the second peripheral cog portion (26).

9. The injection device (100) according to claim 4, wherein the drive cogwheel (221) and the plunger cogwheel (222) are arranged on the same axis (133) or
wherein the drive cogwheel (221) is rotatable with respect to the plunger cogwheel (222) in one direction only.

10. The injection device (100) according to claim 4 or 9, wherein a circumference of the drive cogwheel (221) is larger than a circumference of the plunger cogwheel (222).

11. The injection device (1, 100) according to any one of the preceding claims, wherein the drive rod (11, 111) has an idle position and wherein the injection device further comprises a return spring (35, 135) arranged to force the drive rod towards the idle position.

12. The injection device (1, 100) according to claim 11, further comprising: a housing (8) including a partition between the drive rod and the plunger rod,
wherein the return spring (35) is arranged in the partition so as to act on the drive rod (11) to permit movement between the idle position and a drive position upon applying pressure to the plunger rod (12) in a direction in which substance is expelled from plunger rod (12).

13. The injection device (1, 100) according to claim 11, wherein in the idle position, the drive cogwheel (21) does not engage the plunger cogwheel (22).

14. The injection device (1, 100) according to claim 1, further comprising:
a housing (8) comprising apertures (34) through which the plunger rod (12) is configured to travel in an axial direction, and
a guiding track (36) configured to guide the drive rod (11) in the axial direction.

15. The injection device (1, 100) according to any one of the preceding claims, further comprising a housing (8), wherein at least a portion of the plunger rod (12) is disposed outside the housing (8).

16. The injection device (1, 100) according to any one of the preceding claims, wherein the finger grip (2) houses at least a portion of the drive mechanism (10) and wherein the finger grip (2) comprises two flanges (7) extending in opposite directions from each other, and
the finger grip (2) forms at least a portion of the housing (8).

## Patentansprüche

1. Injektionsvorrichtung (1, 100), die umfasst:
einen Antriebsmechanismus (10, 110), wobei der Antriebsmechanismus umfasst:
eine Antriebsstange (11, 111), die axial beweglich ist;
eine Kolbenstange (12, 112), die axial beweglich ist; und
eine Getriebeanordnung (20, 200),
wobei die Getriebeanordnung derart angeordnet ist, dass eine axiale Bewegung der Antriebsstange eine axiale Bewegung der Kolbenstange erzeugt,
wobei der Antriebsmechanismus (10, 110) an einem Fingergriff (2, 102) der Injektionsvorrichtung angeordnet ist, und
**dadurch gekennzeichnet, dass** der Fingergriff (2, 102) lösbar angeordnet ist.

2. Injektionsvorrichtung (1, 100) nach Anspruch 1, wobei die axiale Bewegung der Antriebsstange (11, 111) eine axiale Bewegung der Kolbenstange (12, 112) in derselben Richtung erzeugt.

3. Injektionsvorrichtung (1, 100) nach Anspruch 1 oder 2, wobei die Antriebsstange (11, 111) eine gekerbte Oberfläche (13, 113) umfasst, und die Kolbenstange (12, 112) eine gekerbte Oberfläche (16, 116) umfasst.

4. Injektionsvorrichtung (1, 100) nach einem der vorhergehenden Ansprüche, wobei die Getriebeanordnung (20, 200) ein Antriebszahnrad (21, 221), das mit der Antriebsstange (11, 111) im Eingriff ist, und ein Kolbenzahnrad (22, 222), das mit der Kolbenstange (12, 112) im Eingriff ist, umfasst, wobei das Antriebszahnrad und das Kolbenzahnrad miteinander verbunden sind.

5. Injektionsvorrichtung (1) nach Anspruch 4, wobei ein Umfang des Antriebszahnrads (21) kleiner als ein Umfang des Kolbenzahnrads (22) ist, wobei sich der Umfang des Kolbenzahnrads im Eingriff mit dem Umfang des Antriebszahnrads befindet.

6. Injektionsvorrichtung (1) nach Anspruch 4 oder 5, wobei das Kolbenzahnrad (22) ein erstes Radelement (23), das mit dem Antriebszahnrad (21) im Eingriff ist, und ein zweites Radelement (25), das mit der Kolbenstange (12) im Eingriff ist, umfasst.

7. Injektionsvorrichtung (1) nach Anspruch 6, wobei das erste Radelement (23) in Bezug auf das zweite Radelement (25) nur in einer Richtung drehbar ist.

8. Injektionsvorrichtung (1) nach Anspruch 6 oder 7, wobei das Kolbenzahnrad (22) einen ersten Umfangszahnradabschnitt (24), der an dem ersten Radelement (23) angeordnet ist, und einen zweiten Umfangszahnradabschnitt (26), der an dem zweiten Radelement (25) angeordnet ist, umfasst,
und
wobei der erste Umfangszahnradabschnitt (24) einen Umfang aufweist, der größer als ein Umfang des zweiten Umfangszahnradabschnitts (26) ist.

9. Injektionsvorrichtung (100) nach Anspruch 4, wobei das Antriebszahnrad (221) und das Kolbenzahnrad (222) auf derselben Achse (133) angeordnet sind, oder
wobei das Antriebszahnrad (221) in Bezug auf das Kolbenzahnrad (222) in nur einer Richtung drehbar ist.

10. Injektionsvorrichtung (100) nach Anspruch 4 oder 9, wobei ein Umfang des Antriebszahnrads (221) größer als ein Umfang des Kolbenzahnrads (222) ist.

11. Injektionsvorrichtung (1, 100) nach einem der vorhergehenden Ansprüche, wobei die Antriebsstange (11, 111) eine Leerlaufposition aufweist, und wobei die Injektionsvorrichtung ferner eine Rückstellfeder (35, 135) umfasst, die angeordnet ist, um die Antriebsstange in die Leerlaufposition zu drücken.

12. Injektionsvorrichtung (1, 100) nach Anspruch 11, die ferner umfasst: ein Gehäuse (8), das eine Trennung zwischen der Antriebsstange und der Kolbenstange enthält,
wobei die Rückstellfeder (35) in der Trennung so angeordnet ist, um auf die Antriebsstange (11) einzuwirken, um eine Bewegung zwischen der Leerlaufposition und einer Antriebsposition beim Anwenden von Druck auf die Kolbenstange (12) in einer Richtung zu ermöglichen, in der eine Substanz aus der Kolbenstange (12) herausgestoßen wird.

13. Injektionsvorrichtung (1, 100) nach Anspruch 11, wobei in der Leerlaufposition das Antriebszahnrad (21) nicht in das Kolbenzahnrad (22) eingreift.

14. Injektionsvorrichtung (1, 100) nach Anspruch 1, die ferner umfasst:
ein Gehäuse (8), das Öffnungen (34) umfasst, die so konfiguriert sind, dass die Kolbenstange (12) in eine axiale Richtung hindurchfahren kann, und
eine Führungsspur (36), die so konfiguriert ist, dass sie die Antriebsstange (11) in die axiale Richtung führt.

15. Injektionsvorrichtung (1, 100) nach einem der vorhergehenden Ansprüche, die ferner ein Gehäuse (8) umfasst, wobei mindestens ein Abschnitt der Kolbenstange (12) außerhalb des Gehäuses (8) angeordnet ist.

16. Injektionsvorrichtung (1, 100) nach einem der vorhergehenden Ansprüche, wobei der Fingergriff (2) mindestens einen Abschnitt des Antriebsmechanismus (10) aufnimmt, und wobei der Fingergriff (2) zwei Flansche (7) umfasst, die sich in entgegengesetzte Richtungen voneinander erstrecken, und
wobei der Fingergriff (2) mindestens einen Abschnitt des Gehäuses (8) bildet.

## Revendications

1. Dispositif d'injection (1, 100) comprenant :
un mécanisme d'entraînement (10, 110), le mécanisme d'entraînement comprenant :
une tige d'entraînement (11, 111) axialement mobile ;
une tige de piston (12, 112) axialement mobile ; et
un ensemble d'engrenage (20, 200),
dans lequel l'ensemble d'engrenage est agencé de telle sorte qu'un mouvement axial de la tige d'entraînement génère un mouvement axial de la tige de piston,
dans lequel le mécanisme d'entraînement (10, 110) est agencé au niveau d'une prise pour doigt (2, 102) du dispositif d'injection, et
**caractérisé en ce que** la prise pour doigt (2, 102) est agencée de manière amovible.

2. Dispositif d'injection (1, 100) selon la revendication 1, dans lequel le mouvement axial de la tige d'entraînement (11, 111) génère un mouvement axial de la tige de piston (12, 112) dans la même direction.

3. Dispositif d'injection (1, 100) selon la revendication 1 ou 2, dans lequel la tige d'entraînement (11, 111) comprend une surface rainurée (13, 113) et la tige de piston (12, 112) comprend une surface rainurée (16, 116).

4. Dispositif d'injection (1, 100) selon l'une quelconque des revendications précédentes, dans lequel l'ensemble d'engrenage (20, 200) comprend une roue dentée d'entraînement (21, 221) en prise avec la tige d'entraînement (11, 111), et une roue dentée de piston (22, 222) en prise avec la tige de piston (12, 112), la roue dentée d'entraînement et la roue dentée de piston étant reliées l'une à l'autre.

5. Dispositif d'injection (1) selon la revendication 4, dans lequel une circonférence de la roue dentée d'entraînement (21) est plus petite qu'une circonférence de la roue dentée de piston (22), la circonférence de la roue dentée de piston étant en prise avec la circonférence de la roue dentée d'entraînement.

6. Dispositif d'injection (1) selon la revendication 4 ou 5, dans lequel la roue dentée de piston (22) comprend un premier élément de roue (23) en prise avec la roue dentée d'entraînement (21), et un deuxième élément de roue (25) en prise avec la tige de piston (12).

7. Dispositif d'injection (1) selon la revendication 6, dans lequel le premier élément de roue (23) peut être amené en rotation par rapport au deuxième élément de roue (25) dans une direction uniquement.

8. Dispositif d'injection (1) selon la revendication 6 ou 7, dans lequel la roue dentée de piston (22) comprend une première partie de dent périphérique (24) agencée au niveau du premier élément de roue (23), et une deuxième partie de dent périphérique (26) agencée au niveau du deuxième élément de roue (25), et
dans lequel la première partie de dent périphérique (24) présente une circonférence qui est plus grande qu'une circonférence de la deuxième partie de dent périphérique (26) .

9. Dispositif d'injection (100) selon la revendication 4, dans lequel la roue dentée d'entraînement (221) et la roue dentée de piston (222) sont agencées sur le même axe (133) ou dans lequel la roue dentée d'entraînement (221) peut être amenée en rotation par rapport à la roue dentée de piston (222) dans une direction uniquement.

10. Dispositif d'injection (100) selon la revendication 4 ou 9, dans lequel une circonférence de la roue dentée d'entraînement (221) est plus grande qu'une circonférence de la roue dentée de piston (222).

11. Dispositif d'injection (1, 100) selon l'une quelconque des revendications précédentes, dans lequel la tige d'entraînement (11, 111) présente une position inactive et dans lequel le dispositif d'injection comprend en outre un ressort de rappel (35, 135) agencé pour pousser la tige d'entraînement en direction de la position inactive.

12. Dispositif d'injection (1, 100) selon la revendication 11, comprenant en outre : un boîtier (8) comportant une cloison entre la tige d'entraînement et la tige de piston, dans lequel le ressort de rappel (35) est agencé dans la cloison de manière à agir sur la tige d'entraînement (11) pour permettre un mouvement entre la position inactive et une position d'entraînement lors de l'application d'une pression sur la tige de piston (12) dans une direction dans laquelle une substance est expulsée de la tige de piston (12).

13. Dispositif d'injection (1, 100) selon la revendication 11, dans lequel dans la position inactive, la roue dentée d'entraînement (21) n'est pas en prise avec la roue dentée de piston (22).

14. Dispositif d'injection (1, 100) selon la revendication 1, comprenant en outre :
un boîtier (8) comprenant des ouvertures (34) à travers lesquelles la tige de piston (12) est configurée pour se déplacer dans une direction axiale, et
une piste de guidage (36) configurée pour guider la tige d'entraînement (11) dans la direction axiale.

15. Dispositif d'injection (1, 100) selon l'une quelconque des revendications précédentes, comprenant en outre un boîtier (8), dans lequel au moins une partie de la tige de piston (12) est disposée à l'extérieur du boîtier (8).

16. Dispositif d'injection (1, 100) selon l'une quelconque des revendications précédentes, dans lequel la prise pour doigt (2) loge au moins une partie du mécanisme d'entraînement (10) et dans lequel la prise pour doigt (2) comprend deux rebords (7) s'étendant dans des directions opposées l'une à l'autre, et
la prise pour doigt (2) forme au moins une partie du boîtier (8).
